# EUROPEAN PATENT APPLICATION

(11) **EP 1 930 004 A1**
(43) Date of publication of application: **11.06.2008**
(21) Application number: 06025424.0
(22) Date of filing: 08.12.2006
(51) Int. Cl.: A61K 31/427

(54) **Use of epothilones in the treatment of osteoporosis and related diseases**

(71) Applicant: Bayer Schering Pharma Aktiengesellschaft, 13353 Berlin (DE)
(72) Inventor: Kaekoenen, Sanna-Maria, Dr., 13353 Berlin (DE); Hoffmann, Jens, Dr., 16567 Mühlenbeck (DE); Klar, Ulrich, Dr., 13503 Berlin (DE)

(57) **Abstract**

The present invention relates to the use of natural or synthetic Epothilones for the treatment or prophylaxis of diseases caused by a dysbalance of osteoclast and osteoblast activity, especially osteoporosis.

## Description

The present invention relates to the use of natural or synthetic Epothilones for the treatment or prophylaxis of diseases caused by a dysbalance of osteoclast and osteoblast activity, especially osteoporosis.

### Background of the invention

Osteoporosis and related bone diseases are a common disease in the modern society (Sambrook et al., Osteoporosis, Lancet 2006 Jun 17, 367(9527):2001-8). These diseases may be induced by age, by other diseases or they may be a side effect of drug therapies with existing drugs (e.g. Statins, aromatase inhibitors).

A number of treatments has been suggested, including bisphosphonate therapy, hormone therapy, parathyroid therapy.
However, these drug treatments cause significant side effects (e.g. osteonecrosis caused by bisphosphonates (Bornstein et al., Schweiz Monatsschr. Zahnmed., 2006; 116 (10), 1035-1047).

There is therefore still need for new drugs for the treatment and prophylaxis of osteoporosis and related diseases.

### Description of the Invention

The present invention relates to the use of Epothilones in the treatment osteoporosis and related diseases.

The epothilones represent a new class of microtubule stabilizing cytotoxic agents (see Gerth, K. et al., J. Antibiot., 1996, 49, 560-3; or Hoefle et al., Angew. Chem. [Applied Chem.], 1996, 108, 1671-1673). These cytotoxic antimitotic agents, block the mitotic spindle of a proliferating cell by binding to the spindle-peptide tubulin, and thus cause apoptosis (K.-H. Altmann, Curr. Opin. Chem. Biol., 2001, 5, 424-431).

The natural products Epothilone A and B as well as some of their synthetic derivatives have recently found interest in connection with the treatment of cancer, and a lot of work has been done on their synthesis (K. Nicolaou et al., Angew. Chem., 1998, 110, 2120-2153) and the synthesis of modified structures.

WO 99/07692, WO 99/02514, WO 99/67252, and WO 2004/014919 disclose Epothilone derivatives, their synthesis and pharmaceutical use.

WO 00/66589 deals with the synthesis and pharmaceutical use of Epothilone derivatives having an alkenyl-, alkynyl-, or a cyclic ether containing substituent at the 6(10)-position of the macrocyclic ring.

WO 00/49021 discloses Epothilone derivatives with a halogen substituent in the 16(3)-position and their synthesis.

WO 00/71521 discloses a method for the synthesis of olefinic Epothilones.

WO 98/25929 deals with the manufacture of libraries of Epothilone analogs.

WO 99/43320 mentions, in a very general manner, the use of Epothilones for the treatment of cancer.

WO 03/074053 describes the use of Epothilones and Epothilone analogs in the treatment of brain diseases associated with proliferative processes.

WO 04/050089 describes the use of conjugates of Epothilones and Epothilone derivatives (as effectors) with suitable saccharides as saccharide derivatives (as recognition units) in the treatment of proliferative or angiogenesis-associated processes.

WO 2004/012735 describes the use of conjugates of Epothilones and Epothilone derivatives (as effectors) with suitable biomolecules (as recognition units) in the treatment of proliferative or angiogenesis-associated processes.

WO 03/007924 describes the combination of Epothilones with a bisphosphonate, a platinum compound or a vasculostatic compound as use as combination therapy in the treatment of, *inter alia,* bone metastasis.

Finaly, WO 2006/032537 describes the use of Epothilones in the treatment of bone metastasis. Said application does not - however - disclose a treatment of bone diseases in patients without a tumour.

None of these publications, however, describe the use of Epothilones as monotherapy for the treatment of osteoporosis in cancer-free patients. In fact, there is no general or specific disclosure or suggestion in the prior art that pharmaceutically effective amounts of an Epothilone directly decrease osteoclast activity which is the underlying mechanism in the development of osteoporosis and related diseases.
Moreover there is no suggestion in the art that Epothilones in an amount well below the effective amount in cancer treatment may be effective in the treatment of osteoporosis and related diseases.

Thus the technical problem underlying the present invention is to provide compounds for the manufacture of medicaments for use in the treatment osteoporosis and related diseases. The solution to this technical problem is achieved by using Epothilones as described below for the manufacture of said medicaments.

It has now been found that unexpectedly Epothilones can inhibit osteoclast activity, and thus show a beneficial effect in the treatment of osteoporosis and related diseases. Accordingly, the present invention provides the use of an Epothilone in the manufacture of medicaments for use as an inhibitor osteoclast activity and is thus useful for the treatment of osteoporosis and related diseases.

The invention also provides a method of inhibiting osteoclast activity in a patient in need of such treatment, which method comprises the administration of an effective amount of an Epothilone to said patient.

The invention also relates to methods of treating osteoporosis by oral, parenteral, rectal, or local, preferably inhalational, intravenous, or intraperitoneal, most preferably intravenous administration of an Epothilone.

In a particular embodiment of the present invention, the medicament containing the Epothilone is used to treat, prevent, or alleviate osteoporosis or related diseases. The bone disease results from a dysbalance of osteoblast and osteoclast activity cancer elsewhere in the body, especially an activation of osteoclast activity, leading to bone hypodensity.

Further embodiments of the invention are the use of epothilones to treat, prevent or alleviate osteonecrosis, osteoarthrosis, osteochondrosis, osteodystrophia.

Still further embodiments of the invention are the use of epothilones to treat, prevent or alleviate rheumatic diseases, Spondylitis, Lupus and related auto-immune diseases, padget disease, arthrosis, periodontal disease or inflammatory diseases leading to osteoclast hyperactivity.

Another aspect of the invention is the inhibition of inflammatory cells induced by epothilones. A still further aspect of the invention therefore is the use of epothilones to treat, prevent or alleviate T- , B, and NK killer cell mediated diseases.

For the purposes of the present invention, an Epothilone is defined as a cyclic molecule with a 16-membered ring and variable substituents and pharmaceutical activity as a cytostatic agent that binds to tubulin (Asnes et al., Anal. Biochem. 1979, 98, 64-73; Job et al., Cellular Pharmacol. 1993, I (Suppl. I), S7-S10; Lichtner et al., PNAS 2001, 98, 11743-11748). This definition includes natural (especially Epothilone A, Epothilone B (also referred to as EPO-906), Epothilone C, Epothilone D (also referred to as Kos-862), Epothilone E and Epothilone F) as well as semi-synthetic and synthetic Epothilones as decribed in the references cited above and some others. Examples for semi-synthetic and synthetic Epothilones are BMS-247550, BMS-310705, ABJ-879, Fludelone, KOS-1584 and ZK-EPO, the structures of which are in the public domain and well known to the expert in the art.

For the purpose of this application the term "Epothilone" also includes Epothilone conjugates and Epothilone pro-drugs such as those described in WO 04/050089 and WO 2004/012735, DE 10234975 which are herein incorporated by reference.

One preferred group of Epothilones is that wherein the Epothilone molecule contains a lactone or a lactame moiety.

Preferred Epothilones for use in the present invention are compounds of the general formula: 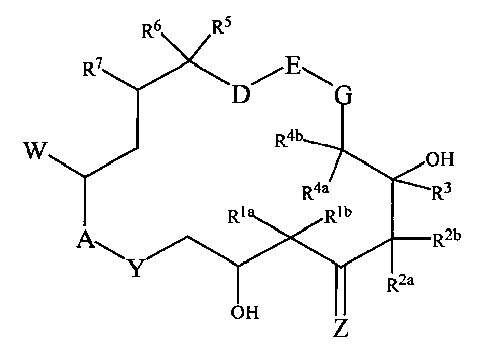 in which
- R^{1a}, R^{1b}: are each independently hydrogen, C₁-C₁₀ alkyl, aryl, aralkyl, or together form a -(CH₂)ₘ-group where m is 2 to 5;
- R^{2a}, R^{2b}: are each independently hydrogen, C₁-C₁₀ alkyl, aryl, aralkyl, or together form a -(CH₂)ₙ-group where n is 2 to 5, or are C₂-C₁₀ alkenyl or C₂-C₁₀ alkynyl;
- R³: is hydrogen, C₁-C₁₀ alkyl, aryl or aralkyl;
- R^{4a}, R^{4b}: are each independently hydrogen, C₁-C₁₀ alkyl, aryl, aralkyl, or together form a -(CH₂)ₚ group where p is 2 to 5;
- R⁵: is hydrogen, C₁-C₁₀ alkyl, aryl, aralkyl, CO₂H, CO₂alkyl, CH₂OH, CH₂Oalkyl, CH₂Oacyl, CN, CH₂NH₂, CH₂NH(alkyl), CH₂N(alkyl)₂, CH₂NH(acyl), CH₂N(acyl)₂ or CH₂Hal;
- R⁶, R⁷: are each hydrogen, or together form an additional bond, or together form an epoxy function;
- G: is O or CH₂;
- D-E: together is a group -H₂C-CH₂-, -HC=CH-, -C≡C-, -CH(OH)-CH(OH)-, -CH(OH)-CH₂-, -CH₂-CH(OH)-, -CH₂-O-, -O-CH₂-, or 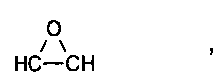 whereby if G is O then D-E is not CH₂-O; or
- D-E-G: together is a group H₂C-CH=CH or a group CH=CH-CH₂;
- W: is a group C(=X)R⁸, or is a bi- or tricyclic aromatic or heteroaromatic radical;
- X: is O,
or two groups OR²⁰
or a C₂-C₁₀ alkylenedioxy group (which may be straight or branched),
or H and the group OR⁹,
or a group CR¹⁰R¹¹;
- R⁸: is hydrogen, C₁-C₁₀ alkyl, aryl, aralkyl, halogen, CN;
- R⁹: is hydrogen or a protecting group PGX;
- R¹⁰, R¹¹: are each independently hydrogen, C₁-C₂₀ alkyl, aryl, aralkyl, or together with the methylene carbon form a 5- to 7-membered carbocyclic ring;
- Z: is O
or H and the group OR¹²;
- R¹²: is hydrogen or a protecting group PGZ;
- A-Y: is a group O-C(=O), O-CH₂, CH₂-C(=O), NR₂₁-C(=O), or NR₂₁-SO₂;
- R²⁰: is a C₁-C₂₀ alkyl group;
- R²¹: is hydrogen, or C₁-C₁₀ alkyl;
- PGx, PGz: is C₁-C₂₀ alkyl, C₄-C₇ cycloalkyl, which may contain one or more oxygen atoms in the ring, aryl, aralkyl, C₁-C₂₀ acyl, aroyl, C₁-C₂₀ alkylsulfonyl, arylsulfonyl, tri(C₁-C₂₀ alkyl)silyl, di(C₁-C₂₀ alkyl) arylsilyl, (C₁-C₂₀ alkyl) diarylsilyl, or tri(aralkyl)silyl;
as a single stereoisomer or a mixture of different stereoisomers, and/or as a pharmaceutically acceptable salt thereof.

Further preferred Epothilones for use in the present invention are compounds of the general formula: 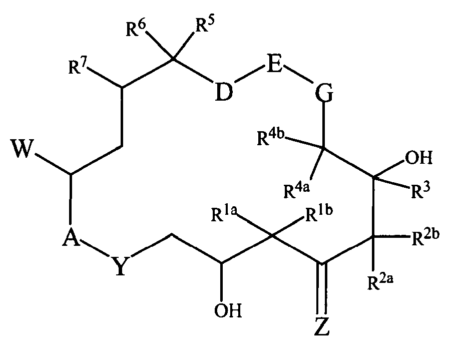 in which
- A-Y: is O-C(=O);
- D-E: is H₂C-CH₂;
- G: is CH₂;
- Z: is O;
- R^{1a}, R^{1b}: are both C₁-C₁₀ alkyl or together form a -(CH₂)ₘ- group where m is 2 or 3;
- R^{2a}, R^{2b}: are each independently hydrogen, C₁-C₁₀ alkyl, C₂-C₁₀ alkenyl, or C₂-C₁₀ alkynyl;
- R³: is hydrogen;
- R^{4a}, R^{4b}: are each independently hydrogen or C₁-C₁₀ alkyl;
- R⁵: is C₁-C₁₀ alkyl.

More preferred Epothiloneas are those in which:
- R^{2a}, R^{2b}: are each independently hydrogen, C₂-C₁₀ alkenyl or C₂-C₁₀ alkynyl;
- R⁶, R⁷: together form an epoxy function or an additional bond; and
- W: is a 2-Methyl-benzothiazol-5-yl radical,
a 2-Methyl-benzoxazol-5-yl radical,
or a Quinoline-7-yl radical.

Such Epothilones are for example:
(4S,7R,8S,9S,13E/Z,16S)-4,8-dihydroxy-16-(2-methyl-benzoxazol-5-yl)-1-oxa-5,5,9,13-tetramethyl-7-(prop-2-en-1-yl)-cyclohexadec-13-ene-2,6-dione;
(1S/R,3S(E),7S,10R,11R,12S,16R/S)-7,11-dihydroxy-10-(prop-2-en-1-yl)-3-(2-methyl-benzoxazol-5-yl)-8,8,12,16-tetramethyl-4,17-dioxabicyclo[14.1.0]heptadecane-5,9-dione;
(4S,7R,8S,9S,13E/Z,16S)-4,8-dihydroxy-16-(2-methyl-benzothiazol-5-yl)-1-oxa-5,5,9,13-tetramethyl-7-(prop-2-en-1-yl)-cyclohexadec-13-ene-2,6-dione;
(1S/R,3S,7S,10R,11R,12S,16R/S)-7,11-dihydroxy-10-(prop-2-en-1-yl)-3-(2-methyl-benzothiazol-5-yl)-8,8,12,16-tetramethyl-4,17-dioxabicyclo[14.1.0]heptadecane-5,9-dione;
(4S,7R,8S,9S,13E/Z,16S)-4,8-dihydroxy-16-(2-methyl-benzothiazol-5-yl)-1-oxa-9,13-dimethyl-5,5-(1,3-trimethylen)-7-(prop-2-en-1-yl)-cyclohexadec-13-ene-2,6-dione;
(1S/R,3S,7S,10R,11R,12S,16R/S)-7,11-dihydroxy-10-(prop-2-en-1-yl)-3-(2-methyl-benzothiazol-5-yl)-12,16-dimethyl-8,8-(1,3-trimethylen)-4,17-dioxabicyclo[14.1.0]heptadecane-5,9-dione;
(4S,7R,8S,9S,13E/Z,16S)-4,8-dihydroxy-16-(2-methyl-benzothiazol-5-yl)-1-oxa-5,5,9,13-tetramethyl-7-(prop-2-in-1-yl)-cyclohexadec-13-ene-2,6-dione;
(1S/R,3S,7S,10R,11R,12S,16R/S)-7,11-dihydroxy-10-(prop-2-in-1-yl)-3-(2-methyl-benzothiazol-5-yl)-8,8,12,16-tetramethyl-4,17-dioxabicyclo[14.1.0]heptadecane-5,9-dione;
(4S,7R,8S,9S,13E/Z,16S)-4,8-dihydroxy-16-(chinolin-7-yl)-1-oxa-5,5,9,13-tetramethyl-7-(prop-2-en-1-yl)-cyclohexadec-13-ene-2,6-dione;
(1S/R,3S,7S,10R,11R,12S,16R/S)-7,11-dihydroxy-10-(prop-2-en-1-yl)-3-(chinolin-2-yl)-8,8,12,16-tetramethyl-4,17-dioxabicyclo[14.1.0] heptadecane-5,9-dione;
(1S,3S,7S,10R,11S,12S,16R)-7,11-dihydroxy-3-(2-methyl-benzothiazol-5-yl)-10-(prop-2-en-1-yl)-8,8,12,16-tetramethyl-4,17-dioxabicyclo[14.1.0] heptadecane-5,9-dione;
(4S,7R,8S,9S,13E/Z,16S)-4,8-dihydroxy-16-(2-methyl-benzothiazol-5-yl)-1-aza-5,5,9,13-tetramethyl-7-(prop-2-en-1-yl)-cyclohexadec-13-ene-2,6-dione;and
(1S/R,3S,7S,10R,11R,12S,16R/S)-7,11-dihydroxy-10-(prop-2-en-1-yl)-3-(2-methyl-benzothiazol-5-yl)-8,8,12,16-tetramethyl-4-aza-17-oxabicyclo[14.1.0]heptadecane-5,9-dione.

Another group of Epothilones which are preferred are Epothilones in which:
- R^{2a}, R^{2b}: are each independently hydrogen, or C₁-C₁₀ alkyl;
- R⁶, R⁷: together form an epoxy function or an additional bond;
- W: is a group C(=X)R⁸;
- X: is a group CR¹⁰R¹¹;
- R8: is hydrogen, halogen, or C₁-C₁₀ alkyl; and
- R¹⁰, R¹¹: are hydrogen/2-methyl-thiazol-4-yl, hydrogen/2-pyridyl, hydrogen/2-methylamine-thiazol-4-yl, or hydrogen/2-methylsulfanyl-thiazol-4-yl.

This group of Epothilones may be exemplified by the following examples:
(4S,7R,8S,9S,13E/Z,16S(E))-4,8-dihydroxy-16-(1-methyl-2-(2-methyl-4-thiazolyl)ethenyl)-1-oxa-5,5,9,13-tetramethyl-7-ethyl-cyclohexadec-13-ene-2,6-dione;
(1S/R,3S(E),7S,10R,11R,12S,16R/S)-7,11-dihydroxy-10-ethyl-3-(1-methyl-2-(2-methyl-4-thiazolyl)ethenyl)-8,8,12,16-tetramethyl-4,17-dioxabicyclo[14.1.0]heptadecane-5,9-dione;
(4S,7R,8S,9S,13E/Z,16S(E))-4,8-dihydroxy-16-(1-methyl-2-(2-methyl-4-thiazolyl)ethenyl)-1-oxa-5,5-(1,3-trimethylen)-9,13-dimethyl-7-ethyl-cyclohexadec-13-ene-2,6-dione;
(1S/R,3S(E),7S,10R,11R,12S,16R/S)-7,11-dihydroxy-10-ethyl-3-(1-methyl-2-(2-methyl-4-thiazolyl)ethenyl)-8,8-(1,3-trimethylen)-12,16-dimethyl-4,17-dioxabicyclo[14.1.0]heptadecane-5,9-dione;
(4S,7R,8S,9S,13E/Z,16S(E))-4,8-dihydroxy-16-(1-methyl-2-(2-methyl-4-thiazolyl)ethenyl)-1-oxa-5,5,9,13-tetramethyl-7-propyl-cyclohexadec-13-ene-2,6-dione;
(1S/R,3S(E),7S,10R,11R,12S,16R/S)-7,11-dihydroxy-10-propyl-3-(1-methyl-2-(2-methyl-4-thiazolyl)ethenyl)-8,8,12,16-tetramethyl-4,17-dioxabicyclo[14.1.0]heptadecane-5,9-dione;
(4S,7R,8S,9S,13E/Z,16S(Z))-4,8-dihydroxy-16-(1-fluor-2-(2-methyl-4-thiazolyl)ethenyl)-1-oxa-5,5,7,9,13-pentamethyl-cyclohexadec-13-ene-2,6-dione;
(1S/R,3S(Z),7S,10R,11S,12S,16R/S)-7,11-dihydroxy-3-(1-fluor-2-(2-methyl-4-thiazolyl)ethenyl)-8,8,10,12,16-pentarnethyl-4,17-dioxabicyclo[14.1.0]heptadecane-5,9-dione;
(4S,7R,8S,9S,13E/Z,16S(Z))-4,8-dihydroxy-16-(1-fluor-2-(2-methyl-4-thiazolyl)ethenyl)-1-oxa-5,5,9,13-tetramethyl-7-ethyl-cyclohexadec-13-ene-2,6-dione;
(1S/R,3S(Z),7S,10R,11S,12S,16R/S)-7,11-dihydroxy-3-(1-fluor-2-(2-methyt-4-thiazolyl)ethenyl)-8,8,12,16-tetramethyl-10-ethyl-4,17-dioxabicyclo[14.1.0]heptadecane-5,9-dione;
(4S,7R,8S,9S,13E/Z,16S(Z))-4,8-dihydroxy-16-(1-fluor-2-(2-methyl-4-thiazolyl)ethenyl)-1-oxa-5,5-(1,3-trimethylen)-7,9,13-trimethyl-cyclohexadec-13-ene-2,6-dione;
(1S/R,3S(Z),7S,10R,11S,12S,16R/S)-7,11-dihydroxy-3-(1-fluor-2-(2-methyl-4-thiazolyl)ethenyl)-8,8-(1,3-trimethylen)-10,12,16-trimethyl-4,17-dioxabicyclo[14.1.0]heptadecane-5,9-dione;
(4S,7R,8S,9S,13E/Z,16S(Z))-4,8-dihydroxy-16-(1-fluor-2-(2-methyl-4-thiazolyl)ethenyl)-1-oxa-5,5-(1,3-trimethylen)-9,13-dimethyl-7-ethyl-cyclohexadec-13-ene-2,6-dione;
(1S/R,3S(Z),7S,10R,11S,12S,16R/S)-7,11-dihydroxy-3-(1-fluor-2-(2-methyl-4-thiazolyl)ethenyl)-8,8-(1,3-trimethylen)-12,16-dimethyl-10-ethyl-4,17-dioxabicyclo[14.1.0]heptadecane-5,9-dione;
(4S,7R,8S,9S,13E/Z,16S(Z))-4,8-dihydroxy-16-(1-chlor-2-(2-methyl-4-thiazolyl)ethenyl)-1-oxa-5,5,7,9,13-pentamethyl-cyclohexadec-13-ene-2,6-dione;
(1S/R,3S(Z),7S,10R,11S,12S,16R/S)-7,11-dihydroxy-3-(1-chlor-2-(2-methyl-4-thiazolyl)ethenyl)-8,8,10,12,16-pentamethyl-4,17-dioxabicyclo[14.1.0]heptadecane-5,9-dione;
(4S,7R,8S,9S,13E/Z,16S(Z))-4,8-dihydroxy-16-(1-chlor-2-(2-methyl-4-thiazolyl)ethenyl)-1-oxa-5,5,9,13-tetramethyl-7-ethyl-cyclohexadec-13-ene-2,6-dione;
(1S/R,3S(Z),7S,10R,11S,12S,16R/S)-7,11-dihydroxy-3-(1-chlor-2-(2-methyl-4-thiazolyl)ethenyl)-8,8,12,16-tetramethyl-10-ethyl-4,17-dioxabicyclo[14.1.0]heptadecane-5,9-dione;
(4S,7R,8S,9S,13E/Z,16S(Z))-4,8-dihydroxy-16-(1-chlor-2-(2-methyl-4-thiazolyl)ethenyl)-1-oxa-5,5,9,13-tetramethyl-7-propyl-cyclohexadec-13-ene-2,6-dione;
(1S/R,3S(Z),7S,10R,11S,12S,16R/S)-7,11-dihydroxy-3-(1-chlor-2-(2-methyl-4-thiazolyl)ethenyl)-8,8,12,16-tetramethyl-10-propyl-4,17-dioxabicyclo[14.1.0]heptadecane-5,9-dione;
(4S,7R,8S,9S,13E/Z.16S(E))-4,8-dihydroxy-16-(1-methy)-2-(2-pyridyl)ethenyl)-1-oxa-5,5,9,13-tetramethyl-7-propyl-cyclohexadec-13-ene-2,6-dione;
(1S/R,3S(E),7S,10R,11R,12S,16R/S)-7,11-dihydroxy-10-propyl-3-(1-methyl-2-(2-pyridyl)ethenyl)-8,8,12,16-tetramethyl-4,17-dioxabicyclo[14.1.0]heptadecane-5,9-dione;
(4S,7R,8S,9S,13E/Z,16S(E))-4,8-dihydroxy-16-(1-methyl-2-(2-pyridyl)ethenyl)-1-oxa-5,5-(1,3-trimethylen)-9,13-dimethyl-7-ethyl-cyclohexadec-13-ene-2,6-dione;
(1S/R,3S(E),7S,10R,11R,12S,16R/S)-7,11-dihydroxy-10-ethyl-3-(1-methyl-2-(2-pyridyl)ethenyl)-8,8-(1,3-trimethylen)-12,16-dimethyl-4,17-dioxabicyclo[14.1.0]heptadecane-5,9-dione;
(45,7R,85,95,13E/Z,165(E))-4,8-dihydroxy-16-(1-methyl-2-(2-pyridyl)ethenyl)-1-oxa-5,5-(1,3-trimethylen)-7,9,13-trimethyl-cyclohexadec-13-ene-2,6-dione;
(1S/R,3S(E),7S,10R,11R,12S,16R/S)-7,11-dihydroxy-3-(1-methyl-2-(2-pyridyl)ethenyl)-8,8-(1,3-trimethylen)-10,12,16-trimethyl-4,17-dioxabicyclo[14.1.0]heptadecane-5,9-dione;
(4S,7R,8S,9S,13E/Z,16S(E))-4,8-dihydroxy-16-(1-methyl-2-(2-pyridyl)ethenyl)-1-oxa-5,5,9,13-tetramethyl-7-propyl-cyclohexadec-13-ene-2,6-dione;
(1S/R,3S(E),7S,10R,11R,12S,16R/S)-7,11-dihydroxy-10-propyl-3-(1-methyl-2-(2-pyridyl)ethenyl)-8,8,12,16-tetramethyl-4,17-dioxabicyclo[14.1.0]heptadecane-5,9-dione;
(4S,7R,8S,9S,13E/Z,16S(Z))-4,8-dihydroxy-16-(1-fluor-2-(2-methyl-4-thiazolyl)ethenyl)-1-oxa-5,5-(1,3-trimethylen)-7,9,13-trimethyl-cyclohexadec-13-ene-2,6-dione;
(1S/R,3S(Z),7S,10R,11S,12S,16R/S)-7,11-dihydroxy-3-(1-fluor-2-(2-methyl-4-thiazolyl)ethenyl)-8,8-(1,3-trimethylen)-10,12,16-dimethyl-4,17-dioxabicyclo[14.1.0]heptadecane-5,9-dione;
(4S,7R,8S,9S,13E/Z,16S(Z))-4,8-dihydroxy-16-(1-chlor-2-(2-methyl-4-thiazolyl)ethenyl)-1-oxa-5,5,9,13-tetramethyl-7-ethyl-cyclohexadec-13-ene-2,6-dione;
(1S/R,3S(Z),7S,10R,11S,12S,16R/S)-7,11-dihydroxy-3-(1-chlor-2-(2-methyl-4-thiazolyl)ethenyl)-8,8,12,16-tetramethyl-10-ethyl-4,17-dioxabicyclo[14.1.0] heptadecane-5,9-dione;
(4S, 7R,8S,9S, 13E/Z, 16S(Z))-4,8-dihydroxy-16-(1-fluor-2-(2-methyl-4-thiazolyl)ethenyl)-1-oxa-5, 5,9,13-tetramethyl-7-ethyl-cyclohexadec-13-ene-2,6-dione; and
(1S/R,3S(Z),7S, 10R, 11S, 12S, 16R/S)-7, 11-dihydroxy-3-(1-fluor-2-(2-methyl-4-thiazolyl)ethenyl)-8,8,12,16-tetramethyl-10-ethyl-4,17-dioxabicyclo[14.1.0] heptadecane-5,9-dione.

A still further preferred group of Epothilones are those in which
- R^{2a}, R^{2b}: are each independently hydrogen, C₂-C₁₀ alkenyl or C₂-C₁₀ alkynyl;
- R⁶, R⁷: together form an epoxy function or an additional bond;
- W: is a group C(=X)R⁸;
- X: is a group CR¹⁰R¹¹;
- R⁸: is hydrogen, halogen, or C₁-C₁₀ alkyl; and
- R¹⁰, R¹¹: are hydrogen/2-methylthiazol-4-yl or hydrogen/2-pyridyl.

Examples of this group are:
(4S,7R,8S,9S,13E/Z,16S(E))-4,8-dihydroxy-16-(1-methy)-2-(2-pyridyl)ethenyl)-1-oxa-5,5,9,13-tetramethyl-7-(prop-2-in-1-yl)-cyclohexadec-13-ene-2,6-dione;
(1S/R,3S(E),7S,10R,11R,12S,16R/S)-7,11-dihydroxy-10-(prop-2-in-1-yl)-3-(2-(2-pyridyl)ethenyl)-8,8,12,16-tetramethyl-4,17-dioxabicyclo[14.1.0]heptadecane-5,9-dione;
(4S,7R,8S,9S,13E/Z,16S(E))-4,8-dihydroxy-16-(1-methyl-2-(2-pyridyl)ethenyl)-1-oxa-5,5,9,13-tetramethyl-7-(prop-2-en-1-yl)-cyclohexadec-13-ene-2,6-dione;
(1S/R,3S(E),7S,10R,11R,12S,16R/S)-7,11-dihydroxy-10-(prop-2-en-1-yl)-3-(1-methyl-2-(2-pyridyl)ethenyl)-8,8,12,16-tetramethyl-4,17-dioxabicyclo[14.1.0] heptadecane-5,9-dione;
(4S,7R,8S,9S,13E/Z,16S(E))-4,8-dihydroxy-16-(1-methyl-2-(2-pyridyl)ethenyl)-1-oxa-5,5,9,13-tetramethyl-7-(but-3-in-1-yl)-cyclohexadec-13-ene-2,6-dione;
(1S/R,3S(E),7S,10R,11R,12S,16R/S)-7,11-dihydroxy-10-(but-3-in-1-yl)-3-(1-methyl-2-(2-pyridyl)ethenyl)-8,8,12,16-tetramethyl-4,17-dioxabicyclo[14.1.0] heptadecane-5,9-dione;
(1S/R,3S(E),7S,10R,11R,12S,16R/S)-7,11-dihydroxy-10-(but-3-en-1-yl)-3-(1-methyl-2-(2-pyridyl)ethenyl)-8,8,12,16-tetramethyl-4,17-dioxabicyclo[14.1.0] heptadecane-5,9-dione;
(1S/R,3S(E),7S,10R,11R,12S,16R/S)-7,11-dihydroxy-10-(but-3-en-1-yl)-3-(2-(2-pyridyl)ethenyl)-8,8,12,16-tetramethyl-4,17-dioxabicyclo[14.1.0] heptadecane-5,9-dione;
(4S,7R,8S,9S,13E/Z,16S(Z))-4,8-dihydroxy-16-(1-fluor-2-(2-methylthiazol-4-yl)ethenyl)-1-oxa-5,5,9,13-tetramethyl-7-(prop-2-in-1-yl)-cyclohexadec-13-ene-2,6-dione;
(1S/R,3S(Z),7S,10R,11R,12S,16R/S)-7,11-dihydroxy-10-(prop-2-in-1-yl)-3-(1-fluor-2-(2-methylthiazol-4-yl)ethenyl)-8,8,12,16-tetramethyl-4,17-dioxabicyclo[14.1.0] heptadecane-5,9-dione;
(4S,7R,8S,9S,13E/Z,16S(Z))-4,8-dihydroxy-16-(1-fluor-2-(2-methylthiazol-4-yl)ethenyl)-1-oxa-5,5,9,13-tetramethyl-7-(prop-2-en-1-yl)-cyclohexadec-13-ene-2,6-dione;and
(1S/R,3S(Z),7S,10R,11R,12S,16R/S)-7,11-dihydroxy-10-(prop-2-en-1-yl)-3-(1-fluor-2-(2-methylthiazol-4-yl)ethenyl)-8,8,12,16-tetramethyl-4,17-dioxabicyclo[14.1.0] heptadecane-5,9-dione.

Another prefered group of Epothilones are those in which
- A-Y: is NR²¹-C(=O).

The most preferred Epothilone is: 7,11-dihydroxy-3-(2-methyl-benzothiazol-5-yl)-10-(prop-2-en-1-yl)-8,8,12,16-tetramethyl-4,17-dioxabicyclo[14.1.0]heptadecane-5,9-dione.

The term "alkyl" as used herein refers to straight or branched alkyl groups, e. g., methyl, ethyl, propyl, isopropyl, n-butyl, t butyl, n-pentyl, neopentyl, heptyl, or decyl. Alkyl groups can be perfluorated or substituted by one to five substituents selected from the group consisting of halogen, hydroxy, C₁-C₄ alkoxy, or C₆-C₁₂ aryl (which can be substituted by one to three halogen atoms).

The term "alkenyl" as used herein refers to a straight or branched chain monovalent or divalent radical, containing at least one double bond and having from two to ten carbon atoms, e.g., ethenyl, prop-2-en-1-yl, but-1-enyl, pent-1-enyl, penta-1,4-dienyl, and the like.

The term "alkynyl" as used herein refers to a substituted or unsubstituted straight or branched chain monovalent or divalent radical, containing at least one triple bond and having from two to ten carbon atoms, e.g., ethynyl, prop-1-ynyl, but-1-ynyl, pent-1-ynyl, pent-3-ynyl, and the like.

Alkenyl and alkenyl groups can be substituted by one or more substituents selected from the group consisting of halogen, hydroxy, alkoxy, -CO₂H, -CO₂Alkyl, -NH₂, -NO₂, -N₃, -CN, C₁-C₂₀ acyl, or C₁-C₂₀ acyloxy.

The term "aryl" as used herein refers to an aromatic carbocyclic or heterocyclic moiety containing five to 14 ring atoms, e.g., phenyl, naphthyl, furyl, thienyl, pyridyl, pyrazolyl, pyrimidinyl, oxazolyl, pyridazinyl, pyrazinyl, chinolyl, or thiazolyl. Aryl groups can be substituted by one or more substituents selected from the group consisting of halogen, hydroxy, alkoxy, -CO₂H, -CO₂Alkyl, -NH₂, Alkyl-NH₂, C₁-C₂₀ alkyl-thiolanyl, -NO₂, -N₃, -CN, C₁-C₂₀ alkyl, C₁-C₂₀ acyl, or C₁-C₂₀ acyloxy. The heteroatoms can be oxidized, if this does not cause a loss of aromatic character, e. g., a pyridine moiety can be oxidized to give a pyridine N-oxide.

The term "aralkyl" as used herein refers to a group which can contain up to 14 atoms in the aryl ring (preferred five to ten) and one to eight carbon atoms in the alkyl chain (preferred one to four), e.g., benzyl, phenylethyl, naphthylmethyl, naphthylethyl, furylmethyl, thienylethyl, or pyridylpropyl. The rings can be substituted by one or more substituents selected from the group consisting of halogen, hydroxy, alkoxy,-CO₂H, -CO₂Alkyl, -NH₂, -NO₂, -N₃, -CN, C₁-C₂₀ alkyl, C₁-C₂₀ acyl, or C₁-C₂₀ acyloxy.

The protecting groups PG can be alkyl- and/or aryl-substituted silyl moieties, C₁-C₂₀ alkyl, C₄-C₇ cycloalkyl, which may contain an oxygen atom in the ring, aryl, aralkyl, C₁-C₂₀ acyl, aroyl, alkyl- or arylsulfonyl. Groups which can be easily be removed from the molecule are preferred, e.g., methoxymethyl, methoxyethyl, polyethylene glycol, ethoxyethyl, tetrahydropyranyl, tetrahydrofuranyl, trimethylsilyl, triethylsilyl, *t-*butyldimethylsilyl, tribenzylsilyl, triisopropylsilyl, benzyl, *p*-nitrobenzyl, *p-*methoxybenzyl, as well as alkylsulfonyl or arylsulfonyl. Preferred acyl groups are formyl, acetyl, propionyl, pivaloyl, butyryl, or benzoyl, which all can be substituted by one or more amino and/or hydroxy moieties.

The synthesis of the compounds listed above is described in the international patent applications WO 99/07692, WO 00/49021, WO 03/041068, WO 03/041063, WO 00/66589, WO 04/050089 and WO 2004/012735 which are incorporated herein by reference.

For the use according to the invention, the compounds can be formulated by methods known in the art. Compositions for the oral, rectal, parenteral or local application can be prepared in the form of tablets, capsules, granulates, suppositories, implantates, sterile injectable aqueous or oily solutions, suspensions or emulsions, aerosols, salves, creams, or gels, retard preparations or retard implantates or even coated catheters or coated stents. The compounds may also be administered by implantable dosing systems.

The pharmaceutical active compound(s) can thus be mixed with adjuvants known in the art, such as gum arabic, talcum, starch, mannitol, methyl cellulose, lactose, surfactants such as tweens® or myrj®, magnesium stearate, aqueous or nonaqueous carriers, paraffin derivatives, wetting agents, dispersing agents, emulsifiers, preservatives, and flavors.

The compounds can be used in the form of their clathrates of α-, β-, or γ-cyclodextrin or of substituted α-, β-, or γ-cyclodextrines, or in the form of a liposomal composition, in particular a liposomal composition comprising a polyethyleneglycol(PEG)-derivatized lipid. The compound may also be used in the form of a nanoformulation.

The term "therapeutically effective amount" as used herein refers to that amount of a compound of the invention which, when administered to an individual in need thereof, is sufficient to effect treatment, as defined below, for osteoporosis and related diseases. The amount which constitutes a "therapeutically effective amount" will vary depending on the compound, the disease and its severity, and the age of the human to be treated, but can be determined routinely by one of ordinary skill in the art having regard to his own knowledge and to this disclosure. It has to be appreciated that generally the "therapeutically effective amount" for a given Epothilone in the treatment of osteoporosis and related diseases is much lower than for the treatment of cancer - including bone metastasis - with the same Epothilone - it may be up to 100 times lower. All tests carried out with epothilones demonstrate that osteoclast activity has significantly been inhibited while no cytotoxic effect could be seen. It is therefore evident that treatment of osteoporosis and related diseases is not accompanied with any known side effect of the used epothilone.

The invention also relates to pharmaceutical compositions containing one or more of the pharmaceutically active compounds listed above, and their use for the treatment and in the methods in accordance with the present invention. Preferably, one dose unit of these compositions contains about 0.0001-10 mg of the pharmaceutically active compound(s). The dosage for the use according to the invention for a human is about 0.0001-10 mg per day; a preferred dosage is about 0.001-7 mg per day; a more preferred dosage is about 0.01-5 mg per day.

Compounds of the present invention have demonstrated positive results in osteoporosis treatment in animal models. The compounds of the present invention can be tested for utility through clinical trials, wherein the compounds are administered to human osteoporosis patients.

### Examples:

The invention is demonstrated in the following examples which are not meant to limit the invention in any way:

### Example 1

The objective of this study is to investigate the effects of selected test compounds selected on resorbing activity of human osteoclasts *in vitro.* Bone resorption is studied using a model where human osteoclast precursor cells derived from bone marrow are cultured for 7 days on bovine bone slices and allowed to differentiate into bone-resorbing osteoclasts. At day 7, the culture medium is changed, the test compounds are added, and the formed mature osteoclasts are allowed to resorb bone in an additional 3-day culture period. Tartrate-resistant acid phosphatase isoform 5b activity (TRACP 5b) is measured from the culture medium collected at day 7 as an index of the number of osteoclasts formed in each well during the differentiation period. After the totally 10 days culture period, C-terminal cross-linked telopeptides of type I collagen (CTX) are quantitated from the culture medium as an index of bone resorption. The results are expressed as the resorption index (CTX at day 10/TRACP 5b at day 7), which describes the mean activity of a single osteoclast. In each test, a baseline group without test compounds is included to obtain a baseline value. E64, an inhibitor of cathepsin enzymes and osteoclastic bone resorption, is added to control cultures to demonstrate that the test system can detect inhibition of bone resorption. The study is approved if the results of the control group are significantly different from the results of the baseline group. The test system can also be modified by adding the test compounds at the beginning of the culture period to study if the test compounds affect osteoclast differentiation. Osteoprotegerin is used as a reference inhibitor of osteoclast differentiation in such cultures.

### Test compounds

The primary test compound used in this example is 7,11-dihydroxy-3-(2-methyl-benzothiazol-5-yl)-10-(prop-2-en-1-yl)-8,8,12,16-tetramethyl-4,17-dioxabicyclo[14.1.0]heptadecane-5,9-dione (referred to as EPO-477)

### Control Substances

E64 (catalogue number E3132, obtained from Sigma, St Louis, MO, USA) is used as a reference inhibitor of bone resorption.

Osteoprotegerin (OPG, catalogue number 450-14, obtained from PeproTech EC Ltd, London, UK) is used as a reference inhibitor of osteoclast differentiation, when necessary.

### Description of the test system used

The method of osteoclast culture on bone slices was originally described by Boyde and co-workers (1984) and by Chambers and co-workers (1984). The rate of bone resorption in the cultures was originally determined by counting the number of resorption pits on each bone or dentine slice using a microscope with phase contrast objectives (Sundquist et al. 1990). Later, the pits were visualized using Wheat Germ Agglutinin lectin that specifically binds to the resorbed area in bone (Selander et al. 1994), making it possible to quantitate the total resorbed area using a microscope and computer-assisted image analysis system (Laitala and Väänänen 1994, Hentunen et al. 1995). These methods have two disadvantages: They are time-consuming and they can not detect differences in the depth of the resorption pits, which may cause false results. Pharmatest uses a commercially available method (CrossLaps® for cultures, Nordic Bioscience, Herlev, Denmark) to detect the amount of bone collagen degradation products released into the culture medium as an index of bone resorption (Bagger et al., 1999). This method is rapid and sensitive, and it is a reliable parameter of total resorbed volume (including depth of pits).

Used herein is a method where osteoclast precursor cells derived from human bone marrow are cultured on bovine bone slices. The cells are first allowed to differentiate into mature bone-resorbing osteoclasts, and the formed osteoclasts are then allowed to resorb bone. The system is ideally suitable for determining the effects of test compounds on bone resorption *in vitro.* Test compounds are added into the cell cultures after the differentiation period, and their effect on the resorbing activity of mature osteoclasts is determined.

### References

Bagger YZ, Foged NT, Andersen L, Lou H, Qvist P (1999) CrossLaps for culture: An improved enzyme-linked immunosorbent assay (ELISA) for measuring bone resorption in vitro. J Bone Miner Res 14, Suppl. 1, S370.

Boyde A, Ali NN, Jones SJ (1984) Resorption of dentine by isolated osteoclasts in vitro. Br Dent J 156: 216-220.

Chambers TJ, Revell PA, Fuller K, Athanasou NA (1984) Resorption of bone by isolated rabbit osteoclasts. J Cell Sci 66: 383-399.

Hentunen TA, Lakkakorpi PT, Tuukkanen J, Lehenkari PP, Sampath TK, Väänänen HK (1995) Effects of recombinant human osteogenic protein-1 on the differentiation of osteoclast-like cells and bone resorption. Biochem Biophys Res Commun 209: 433-443.

Laitala T, Väänänen HK (1994) Inhibition of bone resorption in vitro by antisense RNA and DNA molecules targeted against carbonic anhydrase II or two subunits of vacuolar H+-ATPase. J Clin Invest 93: 2311-2318.

Selander K, Lehenkari P, Väänänen HK (1994) The effects of bisphosphonates on the resorption cycle of isolated osteoclasts. Calcif Tissue Int 55: 368-375.

Sundquist K, Lakkakorpi P, Wallmark B, Väänänen HK (1990) Inhibition of osteoclast proton transport by bafilomycin A1 abolishes bone resorption. Biochem Biophys Res Commun 168: 309-313.

### Procedures

### Bone resorption assay

The bone resorption assay is performed according to the above described Standard Operation Procedure. Shortly, human bone marrow-derived stem cells are suspended to culture medium and allowed to attach to bovine bone slices. The bone slices are transferred into 96-well tissue culture plates containing culture medium with appropriate amounts of important growth factors favoring osteoclast differentiation, including M-CSF, RANK-ligand and TGF-beta. The cells are incubated in a CO₂ incubator in humidified atmosphere of 95% air and 5% carbon dioxide at 37°C. At day 7 when osteoclast differentiation is completed, the culture medium is replaced with culture medium containing conditions favoring osteoclast activity, and the test compounds are added. The cell culture is continued for an additional three days, during which the formed mature osteoclasts are allowed to resorb bone in the presence of vehicle, control inhibitor (E64) or test compounds at different concentrations. Tartrate-resistant acid phosphatase isoform 5b activity (TRACP 5b) is measured from the culture medium collected at day 7 using the BoneTRAP® assay (SBA-Sciences, Oulu, Finland). Medium TRACP 5b activity describes the number of osteoclasts formed in each well during the differentiation period. At the end of the culture period, C-terminal cross-linked telopeptides of type I collagen (CTX) released from the bone slices are quantitated as an index of bone resorption using CrossLaps® for culture assay (Nordic Bioscience, Herlev, Denmark). The results are expressed as the resorption index (CTX at day 10/TRACP 5b at day 7), which describes the mean activity of a single osteoclast. When necessary, the ToxiLight® assay (Cambrex, East Rutherford, NJ, USA) is used to detect cytotoxic effects of test compounds.

### Statistical analysis

The mean and standard deviation (SD) of each group is determined. One-way analysis of variance (ANOVA) is used to study if the values obtained between different groups (baseline vs. control and test compounds) are statistically different (with p < 0.05).

### DESCRIPTION OF THE STUDY

This document contains results of preliminary testing of the effects of test compound EPO-477 on differentiation and activity of human osteoclasts. The test compound EPO-477 arrived to Pharmatest from Schering AG as a solid compound. 10 mM stock solution was made by dissolving the compound at 5,43721 mg/ml in 96% EtOH. Appropriate dilutions were made from the stock solution to obtain the desired test concentrations (0,1 nM, 1 nM and 10 nM in osteoclast differentiation assay and 1 nM, 10 nM and 100 nM in osteoclast activity assay).

Two separate experiments were performed in this study, an osteoclast differentiation assay and an osteoclast activity assay. In the osteoclast differentiation assay, osteoclast precursor cells were cultured for 7 days in the presence of the test compound, and TRACP 5b released into the culture medium was determined at day 7 as an index of the number of osteoclasts formed. Cytotoxicity was determined by quantitating the amount of dying cells from the culture medium at day 2. In the osteoclast activity assay, the test compounds were added after osteoclast differentiation was completed at day 7, and the mature osteoclasts were cultured for an additional 3 days, allowing them to resorb bone. Medium TRACP 5b was measured at day 7 to demonstrate the number of osteoclasts present in each well before adding the test compounds. Medium CTX was measured at day 10 to quantitate bone resorption during days 7-10, and the results are expressed as the resorption index CTX at day 10 / TRACP 5b at day 7, which describes the mean resorbing activity of a single osteoclast. Cytotoxicity was determined by quantitating the amount of dying cells from the culture medium at day 10.

One-way analysis of variance (ANOVA) was used to study if the values obtained between different groups were statistically different (with p<0.05). If a statistically significant difference was observed in ANOVA, the results of the test and control groups were compared separately with the results of the baseline group.

### A) Osteoclast differentiation assay

**Table 1:** The effects of test compound EPO-477 on the differentiation of human osteoclasts. The results are shown as medium TRACP 5b activity at day 7. The groups are: BL = Baseline (no added compounds); C = control (100 ng/ml OPG); A1 = 0,1 nM compound EPO-477; A2 = 1 nM compound EPO-477; A3 = 10 nM compound EPO-477.

| Replicate | BL | C | A1 | A2 | A3 |
|---|---|---|---|---|---|
| 1 | 21,91 | 4,46 | 14,72 | 9,20 | 2,88 |
| 2 | 23,66 | 10,67 | 16,43 | 9,20 | 3,02 |
| 3 | 10,40 | 10,53 | 21,41 | 8,25 | 2,34 |
| 4 | 22,06 | 10,19 | 17,57 | 7,59 | 2,15 |
| 5 | 17,71 | 5,70 | 17,37 | 7,52 | 1,93 |
| 6 | 17,46 | 10,00 | 21,95 | 7,84 | 2,41 |
| **Mean** | **18,87** | **8,59** | **18,24** | **8,27** | **2,45** |
| **SD** | **4,85** | **2,76** | **2,85** | **0,77** | **0,42** |

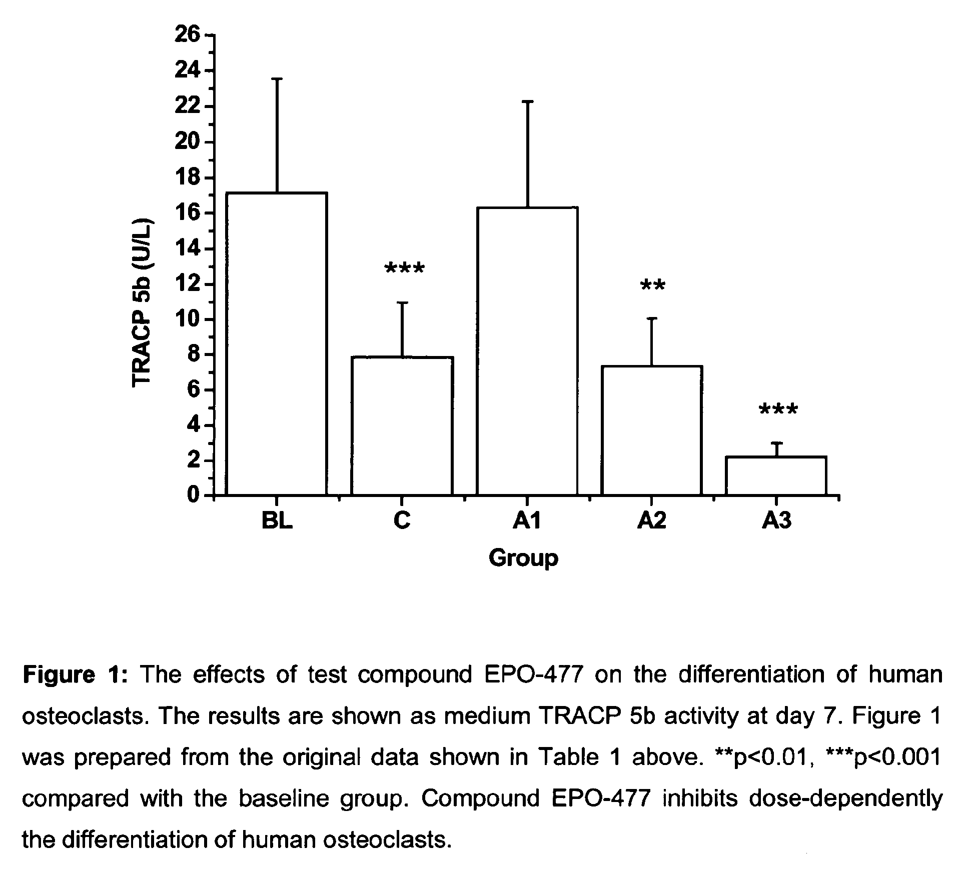

### B) Osteoclast activity assay

**Table 3:** The effects of test compound EPO-477 on the resorbing activity of human osteoclasts. The results are shown as CTX / TRACP 5b values. The CTX values were determined at the end of the resorption period at day 10, and the TRACP 5b values at the beginning of the resorption period at day 7. The groups are: BL = Baseline (no added compounds); C = control (1.0 µM E64); A1 = 1 nM compound EPO-477; A2 = 10 nM compound EPO-477; A3 = 100 nM compound EPO-477. The original CTX values are shown in table 5 and the original TRACP 5b values in table 6.

| Replicate | BL | C | A1 | A2 | A3 |
|---|---|---|---|---|---|
| 1 | 2,78 | 0,41 | 3,32 | 0,79 | 0,87 |
| 2 | 3,17 | 0,64 | 4,36 | 1,45 | 0,92 |
| 3 | 2,74 | 0,41 | 3,20 | 0,84 | 0,56 |
| 4 | 2,84 | 0,38 | 2,98 | 1,00 | 0,89 |
| 5 | 4,01 | 0,59 | 3,09 | 1,93 | 0,36 |
| 6 | 2,62 | 0,29 | 2,90 | 0,82 | 1,07 |
| **Mean** | **3,03** | **0,45** | **3,31** | **1,14** | **0,78** |
| **SD** | **0,51** | **0,13** | **0,53** | **0,46** | **0,26** |

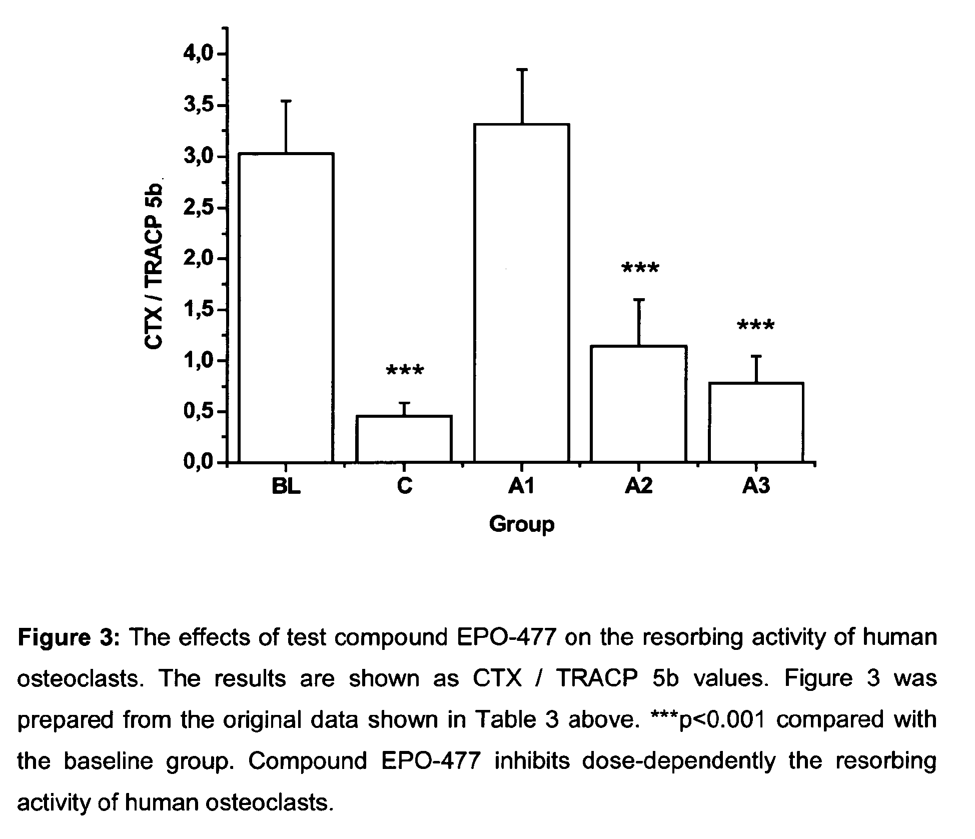

**Table 4:** Cytotoxic effects of test compound EPO-477 in human osteoclast activity assay. The results are shown as the luminescence released from dying cells during the culture period. The groups are: BL = Baseline (no added compounds); C = control (1.0 µM E64); A1 = 1 nM compound EPO-477; A2 = 10 nM compound EPO-477; A3 = 100 nM compound EPO-477.

| Replicate | BL | C | A1 | A2 | A3 |
|---|---|---|---|---|---|
| 1 | 1627 | 1979 | 2371 | 2193 | 2837 |
| 2 | 1697 | 1666 | 1802 | 2097 | 2911 |
| 3 | 1473 | 1489 | 1966 | 2126 | 2428 |
| 4 | 2110 | 1568 | 1781 | 1815 | 2741 |
| 5 | 1573 | 1355 | 1685 | 1909 | 2112 |
| 6 | 1835 | 1507 | 1577 | 1599 | 2964 |
| **Mean** | **1719** | **1594** | **1864** | **1957** | **2666** |
| **SD** | **227** | **214** | **280** | **226** | **331** |

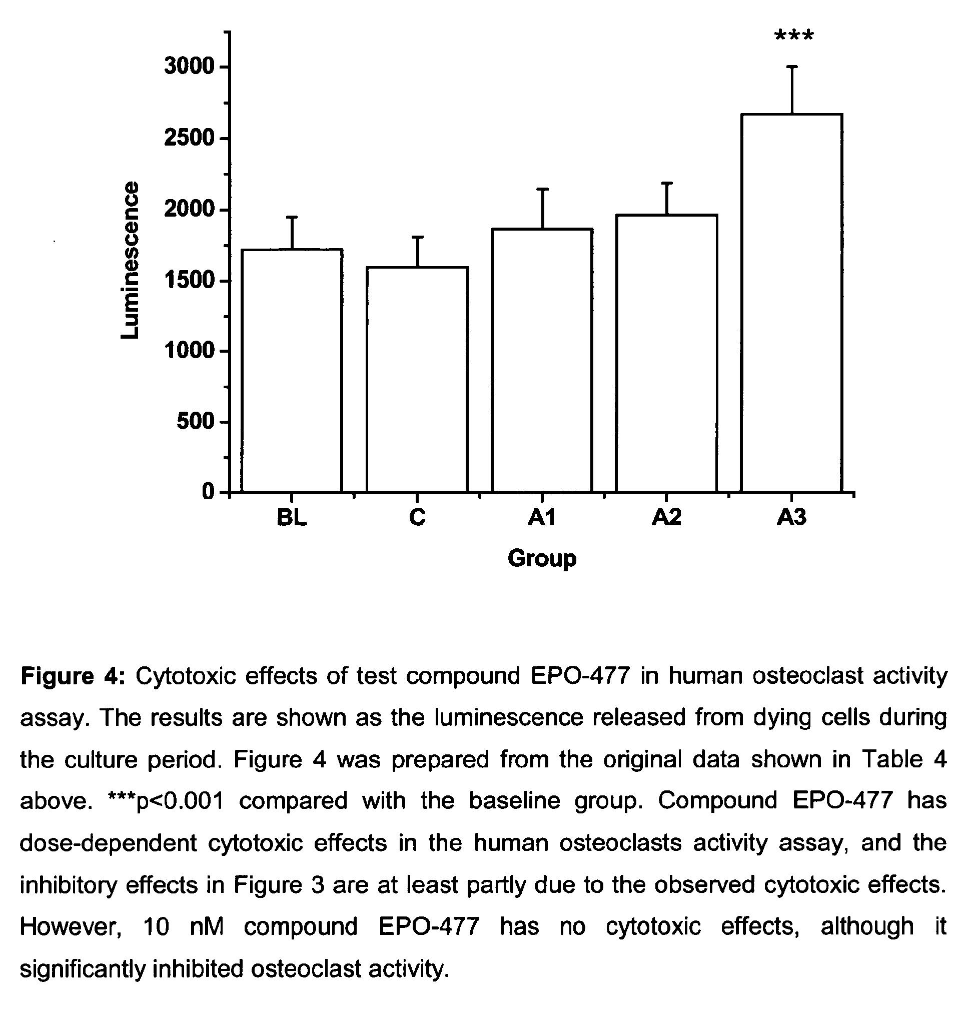

**Table 5:** The effects of test compound EPO-477 on collagen degradation activity of human osteoclasts. The results are shown as the concentration of CTX (nM) released from bone collagen into the culture medium during the resorption phase (days 7-10). The groups are: BL = Baseline (no added compounds); C = control (1.0 µM E64); A1 = 1 nM compound EPO-477; A2 = 10 nM compound EPO-477; A3 = 100 nM compound EPO-477.

| Replicate | BL | C | A1 | A2 | A3 |
|---|---|---|---|---|---|
| 1 | 46,28 | 9,13 | 56,56 | 10,31 | 15,63 |
| 2 | 68,09 | 10,96 | 68,03 | 31,82 | 14,51 |
| 3 | 63,78 | 7,91 | 54,94 | 15,76 | 11,55 |
| 4 | 57,91 | 7,78 | 58,62 | 16,61 | 16,48 |
| 5 | 74,20 | 9,39 | 61,84 | 29,41 | 6,70 |
| 6 | 59,07 | 5,45 | 62,04 | 17,07 | 15,83 |
| **Mean** | **61,56** | **8,44** | **60,34** | **20,16** | **13,45** |
| **SD** | **9,60** | **1,87** | **4,70** | **8,48** | **3,74** |

**Table 6:** Medium TRACP 5b activity (U/L) in each well at the beginning of the resorption phase at day 7. Secreted TRACP 5b activity describes the number of osteoclasts formed in each well during the differentiation period at days 1-7. TRACP 5b was measured to determine the number of osteoclasts in each well before adding the test compounds. The groups are: BL = Baseline (no added compounds); C = control (1.0 µM E64); A1 = 1 nM compound EPO-477; A2 = 10 nM compound EPO-477; A3 = 100 nM compound EPO-477.

| Replicate | BL | C | A1 | A2 | A3 |
|---|---|---|---|---|---|
| 1 | 16,66 | 22,20 | 17,04 | 13,00 | 17,90 |
| 2 | 21,47 | 17,13 | 15,61 | 21,94 | 15,85 |
| 3 | 23,25 | 19,27 | 17,16 | 18,73 | 20,51 |
| 4 | 20,38 | 20,75 | 19,64 | 16,53 | 18,46 |
| 5 | 18,52 | 16,04 | 20,02 | 15,20 | 18,52 |
| 6 | 22,57 | 18,92 | 21,37 | 20,80 | 14,78 |
| **Mean** | **20,48** | **19,05** | **18,47** | **17,70** | **17,67** |
| **SD** | **2,51** | **2,26** | **2,20** | **3,42** | **2,06** |

The above describes results were achieved by using 7,11-dihydroxy-3-(2-methyl-benzothiazol-5-yl)-10-(prop-2-en-1-yl)-8,8,12,16-tetramethyl-4,17-dioxabicyclo[14.1.0]heptadecane-5,9-dione as the test compounds. The use of other epothilones in such tests [including, but not limited to Epothilone A, Epothilone B (also referred to as EPO-906), Epothilone C, Epothilone D (also referred to as Kos-862), Epothilone E and Epothilone F, BMS-247550, BMS-310705, ABJ-879, Fludelone, KOS-1584] shows similar results.

## Claims

1. Use of an Epothilone for the preparation of a medicament for the treatment of diseases associated with a dysbalance of osteoblast and osteoclast activity.

2. Use of an Epothilone for the preparation of a medicament for the treatment of diseases associated with an activation of osteoclast activity.

3. Use of an Epothilone for the in-vitro or in-vivo inhibition of osteoclast activity.

4. Use of an Epothilone for the preparation of a medicament for the treatment or prophylaxis of osteoporosis or related diseases.

5. Use of an Epothilone according to at least one of claims 1-4 for the for the preparation of a medicament for the treatment or prophylaxis of osteoporosis, osteonecrosis, osteoarthrosis, osteochondrosis, osteodystrophia.

6. Use of an Epothilone according to at least one of claims 1-4 for the for the preparation of a medicament for the treatment or prophylaxis of rheumatic diseases, Spondylitis, Lupus and related auto-imune diseases, padget disease, arthrosis, periodontal diesease or inflammatory diseases leading to osteoclast hyperactivity
or T-, B-, and NK killer cell mediated diseases.

7. Use according to any one of claims 1-6, in which the Epothilone is a naturally occuring Epothilone.

8. Use according to any one of claims 1-6, in which the Epothilone is naturally occuring Epothilone A, Epothilone B, Epothilone C, Epothilone D Epothilone E or Epothilone F.

9. Use according to any one of claims 1-6, in which the Epothilone is a semi-synthetic or synthetic Epothilone derivative.

10. Use according to any one of claims 1-9, in which the Epothilone contains a lactone or a lactame moiety.

11. Use according to any one of claims 1-10, in which the Epothilone is a compound of the general formula: 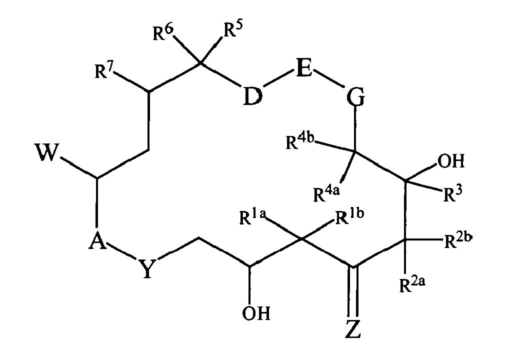 in which
R^{1a}, R^{1b} are each independently hydrogen, C₁-C₁₀ alkyl, aryl, aralkyl, or together form a -(CH₂)ₘ-group where m is 2 to 5;
R^{2a}, R^{2b} are each independently hydrogen, C₁-C₁₀ alkyl, aryl, aralkyl, or together form a -(CH₂)ₙ-group where n is 2 to 5, or are C₂-C₁₀ alkenyl or C₂-C₁₀ alkynyl;
R³ is hydrogen, C₁-C₁₀ alkyl, aryl or aralkyl;
R^{4a}, R^{4b} are each independently hydrogen, C₁-C₁₀ alkyl, aryl, aralkyl, or together form a -(CH₂)ₚ- group where p is 2 to 5;
R⁵ is hydrogen, C₁-C₁₀ alkyl, aryl, aralkyl, CO₂H, CO₂alkyl, CH₂OH, CH₂Oalkyl, CH₂Oacyl, CN, CH₂NH₂, CH₂NH(alkyl), CH₂N(alkyl)₂, CH₂NH(acyl), CH₂N(acyl)₂ or CH₂Hal;
R⁶, R⁷ are each hydrogen, or together form an additional bond, or together form an epoxy function;
G is O or CH₂;
D-E together is a group -H₂C-CH₂-, -HC=CH-, -C≡C-, -CH(OH)-CH(OH)-, -CH(OH)-CH2-, -CH2-CH(OH)-, -CH2-O-, -O-CH₂-, or 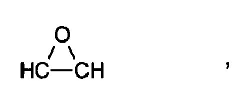 whereby if G is O then D-E is not CH₂-O; or
D-E-G together is a group H₂C-CH=CH or a group CH=CH-CH₂;
W is a group C(=X)R⁸, or is a bi- or tricyclic aromatic or heteroaromatic radical;
X is O,
or two groups OR²⁰,
or a C₂-C₁₀ alkylenedioxy group (which may be straight or branched),
or H and the group OR⁹,
or a group CR¹⁰R¹¹;
R⁸ is hydrogen, C₁-C₁₀ alkyl, aryl, aralkyl, halogen, CN;
R⁹ is hydrogen or a protecting group PGX;
R¹⁰, R¹¹ are each independently hydrogen, C₁-C₂₀ alkyl, aryl, aralkyl, or together with the methylene carbon form a 5- to 7-membered carbocyclic ring;
Z is O
or H and the group OR¹²;
R¹² is hydrogen or a protecting group PGZ;
A-Y is a group O-C(=O), O-CH₂, CH₂-C(=O), NR₂₁-C(=O), or NR₂₁-SO₂;
R²⁰ is a C₁-C₂₀ alkyl group;
R²¹ is hydrogen, or C₁-C₁₀ alkyl;
PGx, PGz is C₁-C₂₀ alkyl, C₄-C₇ cycloalkyl, which may contain one or more oxygen atoms in the ring, aryl, aralkyl, C₁-C₂₀ acyl, aroyl, C₁-C₂₀ alkylsulfonyl, arylsulfonyl, tri(C₁-C₂₀ alkyl)silyl, di(C₁-C₂₀ alkyl) arylsilyl, (C₁-C₂₀ alkyl) diarylsilyl, or tri(aralkyl)silyl;
as a single stereoisomer or a mixture of different stereoisomers, and/or as a pharmaceutically acceptable salt thereof.

12. Use according to claim 11, in which the Epothilone is a compound of the general formula: 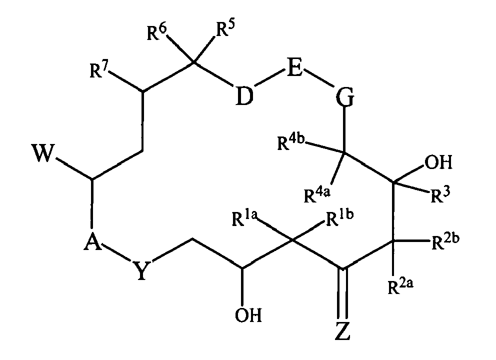 in which
A-Y is O-C(=O);
D-E is H₂C-CH₂;
G is CH₂;
Z is O;
R^{1a}, R^{1b} are both C₁-C₁₀ alkyl or together form a -(CH₂)ₘ- group where m is 2 or 3;
R^{2a}, R^{2b} are each independently hydrogen, C₁-C₁₀ alkyl, C₂-C₁₀ alkenyl, or C₂-C₁₀ alkynyl;
R³ is hydrogen;
R^{4a}, R^{4b} are each independently hydrogen or C₁-C₁₀ alkyl;
R⁵ is C₁-C₁₀ alkyl.

13. Use according to one of claims 11 or 12, wherein
R^{2a}, R^{2b} are each independently hydrogen, C₂-C₁₀ alkenyl or C₂-C₁₀ alkynyl;
R⁶, R⁷ together form an epoxy function or an additional bond; and
W is a 2-Methyl-benzothiazol-5-yl radical,
a 2-Methyl-benzoxazol-5-yl radical,
or a Quinoline-7-yl radical.

14. Use according to one of claims 11 or 12, wherein the compound is selected from the group consisting of:
(4S,7R,8S,9S,13E/Z,16S)-4,8-dihydroxy-16-(2-methyl-benzoxazol-5-yl)-1-oxa-5,5,9,13-tetramethyl-7-(prop-2-en-1-yl)-cyclohexadec-13-ene-2,6-dione;
(1S/R,3S(E),7S,10R,11R,12S,16R/S)-7,11-dihydroxy-10-(prop-2-en-1-yl)-3-(2-methyl-benzoxazol-5-yl)-8,8,12,16-tetramethyl-4,17-dioxabicyclo[14.1.0] heptadecane-5,9-dione;
(4S,7R,8S,9S,13E/Z,16S)-4,8-dihydroxy-16-(2-methyl-benzothiazol-5-yl)-1-oxa-5,5,9,13-tetramethyl-7-(prop-2-en-1-yl)-cyclohexadec-13-ene-2,6-dione;
(1S/R,3S,7S,10R,11R,12S,16R/S)-7,11-dihydroxy-10-(prop-2-en-1-yl)-3-(2-methyl-benzothiazol-5-yl)-8,8,12,16-tetramethyl-4,17-dioxabicyclo[14.1.0]heptadecane-5,9-dione;
(4S,7R,8S,9S,13E/Z,16S)-4,8-dihydroxy-16-(2-methyl-benzothiazol-5-yl)-1-oxa-9,13-dimethyl-5,5-(1,3-trimethylen)-7-(prop-2-en-1-yl)-cyclohexadec-13-ene-2,6-dione;
(1S/R,3S,7S,10R,11R,12S,16R/S)-7,11-dihydroxy-10-(prop-2-en-1-yl)-3-(2-methyl-benzothiazol-5-yl)-12,16-dimethyl-8,8-(1,3-trimethylen)-4,17-dioxabicyclo[14.1.0]heptadecane-5,9-dione;
(4S,7R,8S,9S,13E/Z,16S)-4,8-dihydroxy-16-(2-methyl-benzothiazol-5-yl)-1-oxa-5,5,9,13-tetramethyl-7-(prop-2-in-1-yl)-cyclohexadec-13-ene-2,6-dione;
(1S/R,3S,7S,10R,11R,12S,16R/S)-7,11-dihydroxy-10-(prop-2-in-1-yl)-3-(2-methyl-benzothiazol-5-yl)-8,8,12,16-tetramethyl-4,17-dioxabicyclo[14.1.0]heptadecane-5,9-dione;
(4S,7R,8S,9S,13E/Z,16S)-4,8-dihydroxy-16-(chinolin-7-yl)-1-oxa-5,5,9,13-tetramethyl-7-(prop-2-en-1-yl)-cyclohexadec-13-ene-2,6-dione;
(1S/R,3S,7S,10R,11R,12S,16R/S)-7,11-dihydroxy-10-(prop-2-en-1-yl)-3-(chinolin-2-yl)-8,8,12,16-tetramethyl-4,17-dioxabicyclo[14.1.0] heptadecane-5,9-dione;
(1S,3S,7S,10R,11S,12S,16R)-7,11-dihydroxy-3-(2-methyl-benzothiazol-5-yl)-10-(prop-2-en-1-yl)-8,8,12,16-tetramethyl-4,17-dioxabicyclo[14.1.0] heptadecane-5,9-dione;
(4S,7R,8S,9S,13E/Z,16S)-4,8-dihydroxy-16-(2-methyl-benzothiazol-5-yl)-1-aza-5,5,9,13-tetramethyl-7-(prop-2-en-1-yl)-cyclohexadec-13-ene-2,6-dione; and
(1S/R,3S,7S,10R,11R,12S,16R/S)-7,11-dihydroxy-10-(prop-2-en-1-yl)-3-(2-methyl-benzothiazol-5-yl)-8,8,12,16-tetramethyl-4-aza-17-oxabicyclo[14.1.0]heptadecane-5,9-dione.

15. Use according to one of claims 11 or 12, wherein
R^{2a}, R^{2b} are each independently hydrogen, or C₁-C₁₀ alkyl;
R⁶, R⁷ together form an epoxy function or an additional bond;
W is a group C(=X)R⁸;
X is a group CR¹⁰R¹¹;
R8 is hydrogen, halogen, or C₁-C₁₀ alkyl; and
R¹⁰, R¹¹ are hydrogen/2-methyl-thiazol-4-yl, hydrogen/2-pyridyl, hydrogen/2-methylamine-thiazol-4-yl, or hydrogen/2-methylsulfanyl-thiazol-4-yl .

16. Use according to claim 15, wherein the compound is selected from the group consisting of:
(4S,7R,8S,9S,13E/Z,16S(E))-4,8-dihydroxy-16-(1-methyl-2-(2-methyl-4-thiazolyl)ethenyl)-1-oxa-5,5,9,13-tetramethyl-7-ethyl-cyclohexadec-13-ene-2,6-dione;
(1S/R,3S(E),7S,10R,11R,12S,16R/S)-7,11-dihydroxy-10-ethyl-3-(1-methyl-2-(2-methyl-4-thiazolyl)ethenyl)-8,8,12,16-tetramethyl-4,17-dioxabicyclo[14.1.0] heptadecane-5,9-dione;
(4S,7R,8S,9S,13E/Z,16S(E))-4,8-dihydroxy-16-(1-methyl-2-(2-methyl-4-thiazolyl)ethenyl)-1-oxa-5,5-(1,3-trimethylen)-9,13-dimethyl-7-ethyl-cyclohexadec-13-ene-2,6-dione;
(1S/R,3S(E),7S,10R,11R,12S,16R/S)-7,11-dihydroxy-10-ethyl-3-(1-methyl-2-(2-methyl-4-thiazolyl)ethenyl)-8,8-(1,3-trimethylen)-12,16-dimethyl-4,17-dioxabicyclo[14.1.0]heptadecane-5,9-dione;
(4S,7R,8S,9S,13E/Z,16S(E))-4,8-dihydroxy-16-(1-methyl-2-(2-methyl-4-thiazolyl)ethenyl)-1-oxa-5,5,9,13-tetramethyl-7-propyl-cyclohexadec-13-ene-2,6-dione;
(1S/R,3S(E),7S,10R,11R,12S,16R/S)-7,11-dihydroxy-10-propyl-3-(1-methyl-2-(2-methyl-4-thiazolyl)ethenyl)-8,8,12,16-tetramethyl-4,17-dioxabicyclo[14.1.0] heptadecane-5,9-dione;
(4S,7R,8S,9S,13E/Z,16S(Z))-4,8-dihydroxy-16-(1-fluor-2-(2-methyl-4-thiazolyl)ethenyl)-1-oxa-5,5,7,9,13-pentamethyl-cyclohexadec-13-ene-2,6-dione;
(1S/R,3S(Z),7S,10R,11S,12S,16R/S)-7,11-dihydroxy-3-(1-fluor-2-(2-methyl-4-thiazolyl)ethenyl)-8,8,10,12,16-pentamethyl-4,17-dioxabicyclo[14.1.0] heptadecane-5,9-dione;
(4S,7R,8S,9S,13E/Z,16S(Z))-4,8-dihydroxy-16-(1-fluor-2-(2-methyl-4-thiazolyl)ethenyl)-1-oxa-5,5,9,13-tetramethyl-7-ethyl-cyclohexadec-13-ene-2,6-dione;
(1S/R,3S(Z),7S,10R,11S,12S,16R/S)-7,11-dihydroxy-3-(1-fluor-2-(2-methyl-4-thiazolyl)ethenyl)-8,8,12,16-tetramethyl-10-ethyl-4,17-dioxabicyclo[14.1.0] heptadecane-5,9-dione;
(4S,7R,8S,9S,13E/Z,16S(Z))-4,8-dihydroxy-16-(1-fluor-2-(2-methyl-4-thiazolyl)ethenyl)-1-oxa-5,5-(1,3-trimethylen)-7,9,13-trimethyl-cyclohexadec-13-ene-2,6-dione;
(1S/R,3S(Z),7S,10R,11S,12S,16R/S)-7,11-dihydroxy-3-(1-fluor-2-(2-methyl-4-thiazolyl)ethenyl)-8,8-(1,3-trimethylen)-10,12,16-trimethyl-4,17-dioxabicyclo[14.1.0]heptadecane-5,9-dione;
(4S,7R,8S,9S,13E/Z,16S(Z))-4,8-dihydroxy-16-(1-fluor-2-(2-methyl-4-thiazolyl)ethenyl)-1-oxa-5,5-(1,3-trimethylen)-9,13-dimethyl-7-ethyl-cyclohexadec-13-ene-2,6-dione;
(1S/R,3S(Z),7S,10R,11S,12S,16R/S)-7,11-dihydroxy-3-(1-fluor-2-(2-methyl-4-thiazolyl)ethenyl)-8,8-(1,3-trimethylen)-12,16-dimethyl-10-ethyl-4,17-dioxabicyclo[14.1.0]heptadecane-5,9-dione;
(4S,7R,8S,9S,13E/Z,16S(Z))-4,8-dihydroxy-16-(1-chlor-2-(2-methyl-4-thiazolyl)ethenyl)-1-oxa-5,5,7,9,13-pentamethyl-cyclohexadec-13-ene-2,6-dione;
(1S/R,3S(Z),7S,10R,11S,12S,16R/S)-7,11-dihydroxy-3-(1-chlor-2-(2-methyl-4-thiazolyl)ethenyl)-8,8,10,12,16-pentamethyl-4,17-dioxabicyclo[14.1.0] heptadecane-5,9-dione;
(4S,7R,8S,9S,13E/Z,16S(Z))-4,8-dihydroxy-16-(1-ch!or-2-(2-methy)-4-thiazolyl)ethenyl)-1-oxa-5,5,9,13-tetramethyl-7-ethyl-cyclohexadec-13-ene-2,6-dione;
(1S/R,3S(Z),7S,10R,11S,12S,16R/S)-7,11-dihydroxy-3-(1-chlor-2-(2-methyl-4-thiazolyl)ethenyl)-8,8,12,16-tetramethyl-10-ethyl-4,17-dioxabicyclo[14.1.0] heptadecane-5,9-dione;
(4S,7R,8S,9S,13E/Z,16S(Z))-4,8-dihydroxy-16-(1-chlor-2-(2-methyl-4-thiazolyl)ethenyl)-1-oxa-5,5,9,13-tetramethyl-7-propyl-cyclohexadec-13-ene-2,6-dione;
(1S/R,3S(Z),7S,10R,11S,12S,16R/S)-7,11-dihydroxy-3-(1-chlor-2-(2-methyl-4-thiazolyl)ethenyl)-8,8,12,16-tetramethyl-10-propyl-4,17-dioxabicyclo[14.1.0] heptadecane-5,9-dione;
(4S,7R,8S,9S,13E/Z,16S(E))-4,8-dihydroxy-16-(1-methy)-2-(2-pyridyl)ethenyl)-1-oxa-5,5,9,13-tetramethyl-7-propyl-cyclohexadec-13-ene-2,6-dione;
(1S/R,3S(E),7S,10R,11R,12S,16R/S)-7,11-dihydroxy-10-propyl-3-(1-methyl-2-(2-pyridyl)ethenyl)-8,8,12,16-tetramethyl-4,17-dioxabicyclo[14.1.0]heptadecane-5,9-dione;
(4S,7R,8S,9S,13E/Z,16S(E))-4,8-dihydroxy-16-(1-methyl-2-(2-pyridyl)ethenyl)-1-oxa-5,5-(1,3-trimethylen)-9,13-dimethyl-7-ethyl-cyclohexadec-13-ene-2,6-dione;
(1S/R,3S(E),7S,10R,11R,12S,16R/S)-7,11-dihydroxy-10-ethyl-3-(1-methyl-2-(2-pyridyl)ethenyl)-8,8-(1,3-trimethylen)-12,16-dimethyl-4,17-dioxabicyclo[14.1.0] heptadecane-5,9-dione;
(4S,7R,8S,9S,13E/Z,16S(E))-4,8-dihydroxy-16-(1-methyl-2-(2-pyridyl)ethenyl)-1-oxa-5,5-(1,3-trimethylen)-7,9,13-trimethyl-cyclohexadec-13-ene-2,6-dione;
(1S/R,3S(E),7S,10R,11R,12S,16R/S)-7,11-dihydroxy-3-(1-methyl-2-(2-pyridyl)ethenyl)-8,8-(1,3-trimethylen)-10,12,16-trimethyl-4,17-dioxabicyclo[14.1.0]heptadecane-5,9-dione;
(4S,7R,8S,9S,13E/Z,16S(E))-4,8-dihydroxy-16-(1-methyl-2-(2-pyridyl)ethenyl)-1-oxa-5,5,9,13-tetramethyl-7-propyl-cyclohexadec-13-ene-2,6-dione;
(1S/R,3S(E),7S,10R,11R,12S,16R/S)-7,11-dihydroxy-10-propyl-3-(1-methyl-2-(2-pyridyl)ethenyl)-8,8,12,16-tetramethyl-4,17-dioxabicyclo[14.1.0]heptadecane-5,9-dione;
(4S,7R,8S,9S,13E/Z,16S(Z))-4,8-dihydroxy-16-(1-fluor-2-(2-methyl-4-thiazolyl)ethenyl)-1-oxa-5,5-(1,3-trimethylen)-7,9,13-trimethyl-cyclohexadec-13-ene-2,6-dione;
(1S/R,35(Z),75,10R,11S,12S,16R1S)-7,11-dihydroxy-3-(1-fluor-2-(2-methyl-4-thiazolyl)ethenyl)-8,8-(1,3-trimethylen)-10,12,16-dimethyl-4,17-dioxabicyclo[14.1.0] heptadecane-5,9-dione;
(4S,7R,8S,9S,13E/Z,16S(Z))-4,8-dihydroxy-16-(1-chlor-2-(2-methyl-4-thiazolyl)ethenyl)-1-oxa-5,5,9,13-tetramethyl-7-ethyl-cyclohexadec-13-ene-2,6-dione;
(1S/R,3S(Z),7S,10R,11S,12S,16R1S)-7,11-dihydroxy-3-(1-chlor-2-(2-methyl-4-thiazolyl)ethenyl)-8,8,12,16-tetramethyl-10-ethyl-4,17-dioxabicyclo[14.1.0] heptadecane-5,9-dione;
(4S,7R,8S,9S,13E/Z,16S(Z))-4,8-dihydroxy-16-(1-fluor-2-(2-methyl-4-thiazolyl)ethenyl)-1-oxa-5,5,9,13-tetramethyl-7-ethyl-cyclohexadec-13-ene-2,6-dione; and
(1S/R,3S(Z),7S,10R,111S,12S,16R/S)-7,11-dihydroxy-3-(l-fluor-2-(2-methyl-4-thiazolyl)ethenyl)-8,8,12,16-tetramethyl-10-ethyl-4,17-dioxabicyclo[14.1.0] heptadecane-5,9-dione.

17. Use according to one of claims 11 or 12, wherein
R^{2a}, R^{2b} are each independently hydrogen, C₂-C₁₀ alkenyl or C₂-C₁₀ alkynyl;
R⁶, R⁷ together form an epoxy function or an additional bond;
W is a group C(=X)R⁸;
X is a group CR¹⁰R¹¹;
R⁸ is hydrogen, halogen, or C₁-C₁₀ alkyl; and
R¹⁰, R¹¹ are hydrogen/2-methylthiazol-4-yl or hydrogen/2-pyridyl.

18. The use of claim 17, wherein the compound is selected from the group consisting of:
(4S,7R,8S,9S,13E/Z,16S(E))-4,8-dihydroxy-16-(1-methyl-2-(2-pyridyl)ethenyl)-1-oxa-5,5,9,13-tetramethyl-7-(prop-2-in-1-yl)-cyclohexadec-13-ene-2,6-dione;
(1S/R,3S(E),7S,10R,11R,12S,16R/S)-7,11-dihydroxy-10-(prop-2-in-1-yl)-3-(2-(2-pyridyl)ethenyl)-8,8,12,16-tetramethyl-4,17-dioxabicyclo[14.1.0]heptadecane-5,9-dione;
(4S,7R,8S,9S,13E/Z,16S(E))-4,8-dihydroxy-16-(1-methyl-2-(2-pyridyl)ethenyl)-1-oxa-5,5,9,13-tetramethyl-7-(prop-2-en-1-yl)-cyclohexadec-13-ene-2,6-dione;
(1S/R,3S(E),7S,10R,11R,125,16R/S)-7,11-dihydroxy-10-(prop-2-en-1-yl)-3-(1-methyl-2-(2-pyridyl)ethenyl)-8,8,12,16-tetramethyl-4,17-dioxabicyclo[14.1.0] heptadecane-5,9-dione;
(4S,7R,8S,9S,13E/Z,16S(E))-4,8-dihydroxy-16-(1-methyl-2-(2-pyridyl)ethenyl)-1-oxa-5,5,9,13-tetramethyl-7-(but-3-in-1-yl)-cyclohexadec-13-ene-2,6-dione;
(1S/R,3S(E),7S,10R,11R,12S,16R/S)-7,11-dihydroxy-10-(but-3-in-1-yl)-3-(1-methyl-2-(2-pyridyl)ethenyl)-8,8,12,16-tetramethyl-4,17-dioxabicyclo[14.1.0] heptadecane-5,9-dione;
(1S/R,3S(E),7S,10R,11R,12S,16R/S)-7,11-dihydroxy-10-(but-3-en-1-yl)-3-(1-methyl-2-(2-pyridyl)ethenyl)-8,8,12,16-tetramethyl-4,17-dioxabicyclo[14.1.0] heptadecane-5,9-dione;
(1S/R,3S(E),7S,10R,11R,12S,16R/S)-7,11-dihydroxy-10-(but-3-en-1-yl)-3-(2-(2-pyridyl)ethenyl)-8,8,12,16-tetramethyl-4,17-dioxabicyclo[14.1.0]heptadecane-5,9-dione;
(4S,7R,8S,9S,13E/Z,16S(Z))-4,8-dihydroxy-16-(1-fluor-2-(2-methylthiazol-4-yl)ethenyl)-1-oxa-5,5,9,13-tetramethyl-7-(prop-2-in-1-yl)-cyclohexadec-13-ene-2,6-dione;
(1S/R,3S(Z),7S,10R,11R,12S,16R/S)-7,11-dihydroxy-10-(prop-2-in-1-yl)-3-(1-fluor-2-(2-methylthiazol-4-yl)ethenyl)-8,8,12,16-tetramethyl-4,17-dioxabicyclo[14.1.0] heptadecane-5,9-dione;
(4S,7R,8S,9S,13E/Z,16S(Z))-4,8-dihydroxy-16-(1-fluor-2-(2-methylthiazol-4-yl)ethenyl)-1-oxa-5,5,9,13-tetramethyl-7-(prop-2-en-1-yl)-cyclohexadec-13-ene-2,6-dione; and
(1S/R,3S(Z),7S,10R,11R,12S,16R/S)-7,11-dihydroxy-10-(prop-2-en-1-yl)-3-(1-fluor-2-(2-methylthiazol-4-yl)ethenyl)-8,8,12,16-tetramethyl-4,17-dioxabicyclo[14.1.0] heptadecane-5,9-dione.

19. Use according to one of claims 11 or 12, wherein
A-Y is NR²¹-C(=O).

20. Use according to at least one one of claims 7-19, wherein
the epothilone is present in form of a pro-drug or an antibody conjugate.

21. Use according to at least one one of claims 7-20, wherein
the epothilone is formulated as tablets, capsules, granulates, suppositories, implantates, sterile injectable aqueous or oily solutions, suspensions or emulsions, aerosols, salves, creams, or gels, retard preparations, retard implantates, coated catheters or coated stentsor in which i is contained in an implantable dosing system.

22. A method of treating diseases associated with a dysbalance of osteoblast and osteoclast activity comprising administering to an individual in need thereof a therapeutically effective amount of an Epothilone as defined in any one of claims 7 to 20.

23. A method of treating osteoporosis or related diseases comprising administering to an individual in need thereof a therapeutically effective amount of an Epothilone as defined in any one of claims 7 to 20.

24. A method of treating diseases associated with an activation of osteoclast activity comprising administering to an individual in need thereof a therapeutically effective amount of an Epothilone as defined in any one of claims 7 to 20.

25. A method of treating osteoporosis, osteonecrosis, osteoarthrosis, osteochondrosis or osteodystrophia comprising administering to an individual in need thereof a therapeutically effective amount of an Epothilone as defined in any one of claims 7 to 20.

26. A method of treating treating, preventing or alleviating osteoporosis, osteonecrosis, osteoarthrosis, osteochondrosis or osteodystrophia comprising administering to an individual in need thereof a therapeutically effective amount of an Epothilone as defined in any one of claims 7 to 20.

27. A method of treating rheumatic diseases, Spondylitis, Lupus and related auto-immune diseases, padget disease, arthrosis, periodontal disease inflammatory diseases leading to osteoclast hyperactivity comprising administering to an individual in need thereof a therapeutically effective amount of an Epothilone as defined in any one of claims 7 to 20.

28. The use or the method according to any one of claims 1 to 22, wherein the Epothilone is to be administered orally, parenterally, intravenously; rectally, or locally.
